# EUROPEAN PATENT SPECIFICATION

(11) **EP 1 107 779 B1**
(45) Date of publication and mention of the grant of the patent: **15.01.2003**
(21) Application number: 99940399.1
(22) Date of filing: 24.08.1999
(51) Int. Cl.: A61K 38/21, A61K 39/39, A61P 29/00, A61P 33/02, A61P 37/04

(54) **MEDICAMENTS FOR MANIPULATING T-CELL IMMUNE RESPONSE**
ARZNEIMITTEL ZUR MANIPULATION DER T-ZELL IMMUNANTWORT
MEDICAMENTS POUR MANIPULER LA REPONSE IMMUNE DES LYMPHOCYTES T

(30) Priority: 24.08.1998 GB 9818445
(43) Date of publication of application: 20.06.2001
(73) Proprietor: IMPERIAL COLLEGE INNOVATIONS LIMITED, London SW7 2AZ (GB)
(72) Inventor: FOSTER, Graham Russell, Norfolk place London W2 1PG (GB); THOMAS, Howard Christopher, Norfolk Place London W2 1PG (GB)
(74) Representative: Pilkington, Stephanie Joan
(86) International application number: GB9902786
(87) International publication number: WO00010595

(56) References cited:
- WO-A-95/24212
- WO-A-98/33517
- FOSTER G R ET AL: "Different relative activities of human cell-derived interferon-alpha subtypes: IFN-alpha 8 has very high antiviral potency." JOURNAL OF INTERFERON AND CYTOKINE RESEARCH, (1996 DEC) 16 (12) 1027-33. , XP000876621

## Description

The present invention relates to the use of Interferon-α₂₁ in the preparation of medicaments for the activation of Th2 helper cells, particularly medicaments for use in the treatment of inflammatory disorders such as SLE, Rheumatoid arthritis, inflammatory bowel disease, autoimmune hepatitis and alcoholic hepatitis. In addition, the medicaments can be used to treat vaccine failure or diseases such as leishmania. Methods of up-regulating Th2 helper cell response are also provided.

Type I interferons (IFN) are a family of closely related glycoproteins containing many IFN-α subtypes and one IFN-β subspecies. At least 13 different human IFN-α subtypes have been identified by analysis of human cDNA libraries and by protein analysis of the IFNs produced by stimulated lymphoblastoid cells. The reasons for this heterogeneity are not yet fully known. Previous studies have suggested that all type I IFNs bind to an identical receptor and therefore have identical effects. However, a mutant cell line that responds only to IFN-β and interferon-α₈ but not other IFN-α subtypes has been identified showing that these two IFN subspecies either bind to a different receptor or bind in a different way and may therefore have different effects. Molecular analysis of the human Type I IFN receptor thus suggests that the receptor may be able to distinguish between diferent IFN subtypes.

It has previously been shown that individual IFN subtypes have varying efficiacy against viral infections in different tissue types. Thus, in WO95/24212 it was shown that, for instance, IFN-α₈ was most effective against hepatitis infection in the liver. Subsequently, in WO98/33517, it was shown that individual IFN subtypes, again particularly IFN-α₈, could be used to enhance the T cell immune response.

We have now determined that IFN-α₂₁ can activate Th2 helper T cells. This in turn opens up the possibility of more precise manipulation of the T cell immune response and the ability to treat certain conditions which either require up-regulation of Th2 cells or require Th2 response to compensate for an overactive Th1 response.

Thus, in a first aspect, the present invention provides the use of IFN-α₂₁ in the preparation of a medicament for use in up-regulating the Th2 T cell immune response. In preferred embodiments the medicament will be for use in the treatment of an inflammatory condition such as SLE (systemic lupus erythematosus), Rheumatoid arthritis, inflammatory bowel disease, autoimmune hepatitis and acoholic hepatitis or for alleviating "vaccine failure".

In the context of the present invention "vaccine failure" means that in order to achieve successful vaccination the Th2 T cell response may require stimulation. Thus, the efficacy of certain relatively ineffective vaccines can be enhanced in this way.

In a second aspect the present invention provides the use of IFN-α₂₁ in the preparation of a medicament for use in compensating for an overactive Th1 T cell response. In a preferred embodiment the medicament is for use in the treatment of leishmania.

In further aspects the present invention provides:
i) a pharmaceutical formulation for use in the treatment of an inflammatory condition which comprises IFN-α₂₁, optionally together with one or more pharmaceutically acceptable carriers, excipients or diluents;
ii) a pharmaceutical formulation for use in the treatment of vaccine failure or for use in enhancing a vaccine mediated immune response which comprises IFN-α₂₁, optionally together with one or more pharmaceutically acceptable carriers, excipients or diluents;
iii) a pharmaceutical formulation for use in countering an overactive Th1 T cell response which comprises IFN-α₂₁, optionally together with one or more pharmaceutically acceptable carriers, excipients or diluents.
iv) A product comprising at least one immunogen (preferably in the form of a vaccine) and IFN-α₂₁ as a combined preparation for simultaneous, separate or sequential use in immunising/vaccinating a subject.

The medicaments and/or pharmaceutical formulations described herein may be presented in unit dose forms containing a predetermined amount of active ingredient per dose. The precise dose will of course depend on the condition being treated, the route of administration and the age, weight and condition of the patient.

The medicaments and/or pharmaceutical formulations may include one or more of the following; preserving agents, solubilising agents, stabilising agents, wetting agents, emulsifiers, sweeteners, colourants, odourants, salts, buffers, coating agents or antioxidants. They may also contain other therapeutically active agents.

The medicaments and/or pharmaceutical formulations may be adapted for administration by any appropriate route, for example by the oral (including buccal or sublingual), rectal, nasal, topical (including buccal, sublingual or transdermal), vaginal or parenteral (including subcutaneous, intramuscular, intravenous or intradermal) route. Such formulations may be prepared by any method known in the art of pharmacy, for example by bringing into association the active ingredient with the carrier(s) or excipient(s).

Various routes of administration will now be considered in greater detail:

### (i) Oral Administration

Medicaments and/or pharmaceutical formulations adapted for oral administration may be presented as discrete units such as capsules or tablets; powders or granules; solutions syrups or suspensions in aqueous or non-aqueous liquids; edible foams or whips; or oil-in-water liquid emulsions or water-in-oil liquid emulsions.

Tablets or hard gelatine capsules may comprise lactose, maize starch or derivatives thereof, stearic acid or salts thereof.

Soft gelatine capsules may comprise vegetable oils, waxes, fats, semi-solid, or liquid polyols etc.

Solutions and syrups may comprise water, polyols and sugars. For the preparation of suspensions oils (e.g. vegetable oils) may be used to provide oil-in water or water-in-oil suspensions.

### (ii) Transdermal administration

Medicaments and/or pharmaceutical formulations adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6), 318 (1986).

### (iii) Topical administration

Medicaments and/or pharmaceutical formulations adapted for topical administration may be formulated as ointments, creams, suspensions, lotions, powders, solutions, pastes, gels, sprays, aerosols or oils.

For infections of the eye or other external tissues, for example mouth and skin, the formulations are preferably applied as a topical ointment or cream. When formulated in an ointment, the active ingredient may be employed with either a paraffinic or a water-miscible ointment base. Alternatively, the active ingredient may be formulated in a cream with an oil-in-water cream base or a water-in-oil base.

Pharmaceutical formulations adapted for topical administration to the eye include eye drops wherein the active ingredient is dissolved or suspended in a suitable carrier, especially an aqueous solvent.

Pharmaceutical formulations adapted for topical administration in the mouth include lozenges, pastilles and mouth washes.

### (iv) Rectal administration

Medicaments and/or pharmaceutical formulations adapted for rectal administration may be presented as suppositories or enemas.

### (v) Nasal administration

Medicaments and/or pharmaceutical formulations adapted for nasal administration wherein the carrier is a solid include a coarse powder (e.g. having a particle size for example in the range 20 to 500 microns) which is administered in the manner in which snuff is taken, i.e. by rapid inhalation through the nasal passage from a container of the powder held close up to the nose.

Compositions adapted for nasal administration which use liquid carriers include nasal sprays or nasal drops. These may comprise aqueous or oil solutions of the active ingredient.

Pharmaceutical formulations adapted for administration by inhalation include fine particle dusts or mists which may be generated by means of various types of apparatus, e.g. pressurised aerosols, nebulizers or insufflators. Such apparatus can be constructed so as to provide predetermined dosages of the active ingredient.

### (vi) Vaginal administration

Medicaments and/or pharmaceutical formulations adapted for vaginal administration may be presented as pessaries, tampons, creams, gels, pastes, foams or spray formulations.

### (vii) Parenteral administration

Medicaments and/or pharmaceutical formulations adapted for parenteral administration include aqueous and non-aqueous sterile injectable solutions or suspensions. These may contain anti-oxidants, buffers, bacteriostats and solutes which render the compositions substantially isotonic with the blood of the intended recipient. Other components which may be present in such compositions include water, alcohols, polyols, glycerine and vegetable oils, for example. Compositions adapted for parenteral administration may be presented in unit-dose or multi-dose containers, for example sealed ampoules and vials, and may be stored in a freeze-dried (lyophilized) condition requiring only the addition of a sterile liquid carrier, for example sterile water for injections, immediately prior to use. Extemporaneous injection solutions and suspensions may be prepared from sterile powders, granules and tablets.

Preferred unit dosage formulations are those containing a daily dose or sub-dose or an appropriate fraction thereof, of an active ingredient.

It should be understood that in addition to the ingredients particularly mentioned above, the formulations may also include other agents conventional in the art having regard to the type of formulation in question, for example those suitable for oral administration may include flavouring agents.

The invention will now be described with reference to the following example, which should not be construed as in any way limiting the invention. The example refers to the figures in which:
**FIGURE 1**: shows an analysis of the presence of various marker cytokines over 72hrs after stimulation of peripheral blood mononulear cells by IFN-α₂₁;
**FIGURE 2**: shows ED50s for various IFNαs for induction of CD4+ Th2 cells.

### EXAMPLE 1

### i) Cell Stimulating

Peripheral blood mononuclear cells were extracted from blood samples from healthy volunteers using stranded method. 10⁶ cells /ml were treated in 100 µl aliquots with IL-2 (10 IU/ml), anti-TCR antibody (1 µg/ml) and anti-CD28 antibody (5 µg/ml) plus or minus the appropriate IFN-α subtype for 48-72 hours and then analysed by intracellular staining.

### ii) Intracellular Cytokine Staining

Cells were washed once in PBS (pH 7.2), to which was then added 0.3 ml of 4% Paraformaldehyde in PBS, for 10 min at room temperature. Fixed cells were washed twice in PBS and resuspended in PBN (PBS containing 0.2% BAS and 0.02% NaN3) at 10⁶ cell/ml. For intracellular cytokine staining, fixed cells were incubated with saponin buffer (0.5% saponin from Saponaria, Sigma) in PBN for 15 min at room temperature. Cells were washed twice in saponin buffer and stained with (20 µg/ml) FITC-conjugated mouse anti-human IFN-g, PE-conjugated mouse anti-human IL-4 (Becton Dickinson), PE-conjugated mouse anti-human IL-4 (PharMingen) in saponin buffer for 45 min on ice and washed twice in saponin buffer. For subsequent surface staining cells were incubated with PE- or FITC-conjugated anti-CD4 antibody (DAKO) for 30 min on ice and washed twice saponin buffer and resuspended in PBN.

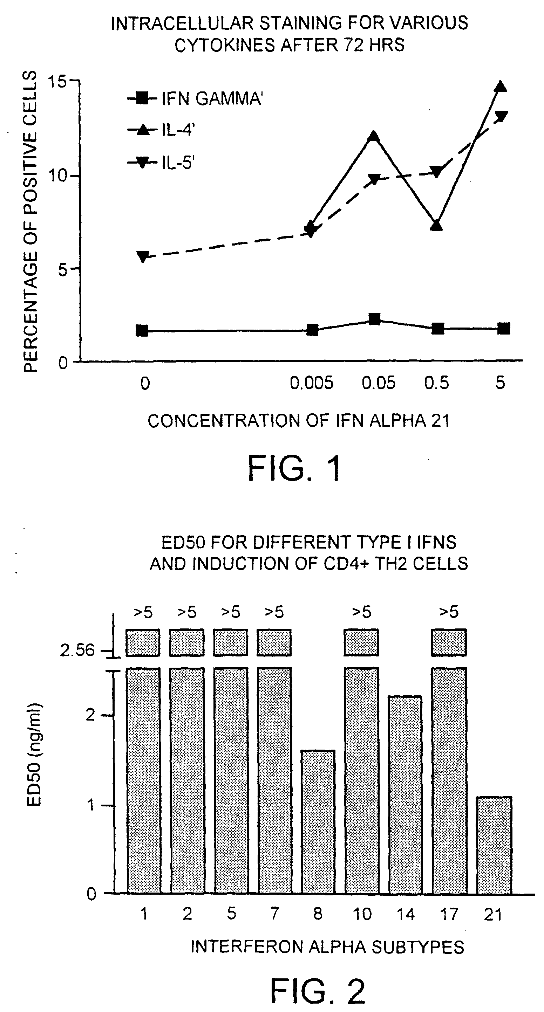

Samples were analyzed on FACSan flow cytometer (Becton Dickinson) equipped with a 480 nm Argon ion laser, 15 mA with a 200 micron nozzle. A minimum of 10,000 cells were counted per sample and the data was analyzed with the Lysis II program (Becton Dickinson). The assays were assessed for markers of Th1 and Th2 cells. The results for IFNα₂₁ are shown in figure 1 while ED50s for various IFNαs are shown if figure 2 for the induction of CD4+ Th2 cells.

Figure 1 clearly shows that IFNα₂₁ stimulates the production of markers of Th2 cells, namely Il-4 and Il-5. In figure 2 it can be seen that most IFN subtypes had no detectable effect on Th2 cells. However IFN subtypes α₈ α₁₄ and α₂₁ had some effect, with IFNα₂₁ being the most potent.

## Claims

1. The use of IFN-α₂₁ in the preparation of a medicament for use in up-regulating the Th2 T cell immune response.

2. The use as claimed in claim 1 wherein the medicament is for use in the treatment of an inflammatory condition such as SLE (systemic lupus erythematosus), Rheumatoid arthritis, inflammatory bowl disease, autoimmune hepatitis and alcoholic hepatitis or for alleviating "vaccine failure".

3. The use of IFN-α₂₁ in the preparation of a medicament for use in compensating for an overactive Th1 T cell response.

4. The use as claimed in claim 3 wherein the medicament is for use in the treatment of leishmania.

5. A pharmaceutical formulation comprising IFN-α₂₁ optionally together with one or more pharmaceutically acceptable carriers, excipients or diluents for use as a medicament.

6. A pharmaceutical formulation as defined in claim 5 for use in the treatment of an inflammatory condition, in the treatment of vaccine failure or for use in enhancing a vaccine mediated immune response, or for countering an overactive Th1 T cell response.

7. A product comprising at least one immunogen (preferably in the form of a vaccine) and IFN-α₂₁ as a combined preparation for simultaneous, separate or sequential use in immunising/vaccinating a subject.

## Revendications

1. Utilisation d'IFN-α₂₁ dans la préparation d'un médicament destiné à être utilisé pour la régulation positive de la réponse immunitaire des cellules T de type Th2.

2. Utilisation selon la revendication 1, dans laquelle le médicament est destiné à être utilisé dans le traitement d'un état inflammatoire comme le lupus érythémateux systémique (LES), l'arthrite rhumatoïde, les maladies inflammatoires digestives, l'hépatite autoimmune et l'hépatite alcoolique ou pour atténuer un "échec vaccinal".

3. Utilisation d'IFN-α₂₁ dans la préparation d'un médicament destiné à être utilisée pour compenser une réponse suractive des cellules T de type Th1.

4. Utilisation selon la revendication 3, dans laquelle le médicament est destiné à être utilisé pour le traitement de la leishmaniose.

5. Formulation pharmaceutique comprenant de l'IFN-α₂₁, facultativement conjointement avec un ou plusieurs véhicules, excipients ou diluants pharmaceutiquement acceptables, destinée à être utilisée comme médicament.

6. Formulation pharmaceutique selon la revendication 5, destinée à être utilisée dans le traitement d'un état inflammatoire, dans le traitement d'un échec vaccinal ou destinée à être utilisée pour améliorer une réponse immune médiée par vaccin ou pour compenser une réponse suractive des cellules T de type Th1.

7. Produit comprenant au moins un immunogène (de préférence sous la forme d'un vaccin) et de l'IFN-α₂₁ en tant que préparation combinée pour une utilisation simultanée, distincte ou séquentielle pour l'immunisation/vaccination d'un sujet.

## Patentansprüche

1. Verwendung von IFN-α₂₁ bei der Herstellung eines Medikaments zur Verwendung für die Hochregulation der Immunantwort von Th2-T-Zellen.

2. Verwendung, wie sie im Anspruch 1 beansprucht wird, wobei das Medikament der Verwendung bei der Behandlung einer entzündlichen Erkrankung, wie des SLE (systemischer Lupus erythematodes), der rheumatoiden Arthritis, der entzündlichen Darmerkrankung, der Autoimmun-Hepatitis oder der alkoholischen Hepatitis, dient sowie dafür eingesetzt wird, dem Fehlen der Wirksamkeit eines Impfstoffs entgegen zu wirken.

3. Verwendung von IFN-α21 bei der Herstellung eines Medikaments zur Neutralisierung einer überschießenden Immunantwort von Th1-T-Zellen.

4. Verwendung, wie sie im Anspruch 3 beansprucht wird, wobei das Medikament der Behandlung der Leishmaniase dient.

5. Pharmazeutische Zusammensetzung, die IFN-α₂₁, gegebenenfalls zusammen mit einem oder mehreren pharmazeutischen Träger(n), Hilfsstoff(en) oder Verdünnungsmittel(n), aufweist, zur Verwendung als Medikament.

6. Pharmazeutische Zusammensetzung, wie sie im Anspruch 5 definiert wird, zur Verwendung bei der Behandlung einer entzündlichen Erkrankung, bei der Behandlung des Fehlens einer Wirksamkeit eines Impfstoffs oder zur Verwendung bei der Verstärkung einer Impfstoff-vermittelten Immunantwort oder zur Neutralisierung einer überschießenden Immunantwort von Th1-T-Zellen.

7. Produkt, dass wenigstens ein Immunogen (vorzugsweise in Form eines Impfstoffs) und IFN-α₂₁ aufweist, als kombiniertes Präparat für die gleichzeitige, getrennte oder sequenzielle Verwendung bei der Immunisierung/Impfung eines Subjekts.
